# EUROPEAN PATENT SPECIFICATION

(11) **EP 1 665 986 B1**
(45) Date of publication and mention of the grant of the patent: **03.06.2009**
(21) Application number: 05111371.0
(22) Date of filing: 28.11.2005
(51) Int. Cl.: A61B 5/154, A61B 5/155

(54) **Blood collection set with an expanded internal volume**
Blutentnahmeset mit vergrössertem Innenvolumen
Ensemble de collecte de sang avec un volume interne accru

(30) Priority: 29.11.2004 US 631515 P; 16.11.2005 US 280622
(43) Date of publication of application: 07.06.2006
(73) Proprietor: Becton, Dickinson and Company, Franklin Lakes, New Jersey 07417-1880 (US)
(72) Inventor: Bennett, Michael C., Summit, NJ 07901 (US); Conway, Hugh T., Verona, NJ 07044 (US)
(74) Representative: Weber, Thomas

(56) References cited:
- EP-A- 1 442 705
- EP-A- 1 602 329
- WO-A-20/04062499
- US-B1- 6 261 263

## Description

### 1. Field of the Invention

The subject invention relates to a blood collection set with an internal chamber, which increases the internal volume of the blood collection set thereby improving flash.

### 2. Description of the Related Art

Phlebotomy procedures often are carried out using a blood collection set. A typical blood collection set includes an IV needle assembly with an IV cannula that has a proximal end, a sharply pointed distal end and a lumen extending between the ends. The needle assembly also includes a plastic IV hub with a proximal end, a distal end, and a passage extending between the ends. The proximal end of the IV cannula is mounted in the passage of the IV hub so that the lumen through the IV cannula communicates with the passage through the IV hub. The needle assembly may further include a shield for shielding the IV cannula after use (a so called safety blood collection set) and a packaging cover for safely covering the IV cannula prior to use. Packaging covers typically are rigid tubes with a proximal end that can be telescoped over the IV cannula and frictionally engaged with the distal end of the IV hub. Shields for shielding the IV cannula of blood collection sets have taken many forms. Some shields are telescoped over the IV hub and can be moved from a proximal position where the cannula is exposed to a distal position where the cannula is shielded. Other shields are hinged to the IV hub and can be rotated from an open position where the IV cannula is exposed to a closed position where the IV cannula is shielded. A needle assembly for a blood collection set also may include two flexible wings that project transversely from the IV hub or from the shield. The wings can be folded into face-to-face relationship with one another to effectively define a handle that facilitates manipulation of the needle assembly. The wings then can be rotated away from one another and held against the skin of the patient.

Blood collection sets also include a length of flexible plastic tubing. The tubing has a distal end that is connected to the proximal end of the IV hub, The tubing also has a proximal end that is connected to a plastic fitting. Thus, fluid communication is provided between the lumen of the IV cannula and the plastic fitting at the proximal end of the flexible tubing. The plastic fitting may be a female luer fitting that can be connected to a male luer fitting. The fitting then can be placed in communication with a reservoir or container for collecting a sample of blood.

Phlebotomy procedures often employ evacuated tubes, such as the VACUTAINER® brand of evacuated tubes sold by Becton Dickinson and Company. Evacuated tubes often are used with a tube holder that has a proximal end, a distal end, and a tubular side wall extending between the ends. The proximal end of the holder is widely open and is configured for slidably receiving the evacuated tube. The distal end of the holder typically includes an end wall with a mounting aperture. The mounting aperture includes internal threads or other mounting structures.

The tube holder may be used with a non-patient needle assembly that has a non-patient hub with external surface configurations for mounting in the mounting aperture of the holder. The non-patient needle assembly further includes a non-patient cannula extending proximally from the hub and a multiple sample sleeve telescoped over the non-patient cannula and mounted to the proximal end of the hub. The hub of the non-patient needle assembly can be threaded or otherwise engaged in the mounting aperture of the tube holder so that the non-patient needle and the multiple sample sleeve project into the tube receiving chamber of the holder.

The blood collection set may be used by mounting the fitting at the proximal end of the flexible plastic tubing to the distal end of the hub of the non-patient needle assembly. The packaging shield that covers the non-patient cannula then may be removed, and the hub of the non-patient needle assembly may be engaged with the tube holder. The medical practitioner then grips the IV needle assembly and removes the packaging cover from the IV cannula. The gripping of the IV needle assembly may include folding the flexible wings into face-to-face engagement and gripping the folded wings between a thumb and forefinger. The pointed distal end of the IV cannula then is urged into a targeted blood vessel. The wings then may be folded into engagement with the skin of the patient and may be taped in position. An evacuated tube then is urged into the open proximal end of the blood collection tube holder so that the non-patient needle pierces the stopper of the evacuated tube. As a result, the blood vessel of the patient is placed in communication with the interior of the evacuated tube, and the pressure differential between the blood vessel and the evacuated tube will generate a flow of blood through the IV cannula, through the passage of the IV hub, through the flexible tubing, through the non-patient hub and finally through the non-patient needle and into the evacuated tube.

The translucent or opaque plastic material of blood collection sets and IV cannula shields, combined with numerous cannula shield mechanism components and the length of the typical needle hub tends to inhibit a clear indication of venous or arterial access. Blood flow into the plastic tubing does provide an indication of venous or arterial access (known as "flash") as the plastic tubing is often formed from a highly translucent or transparent plastic material. However, because of the existence of the elements associated with the IV needle assembly the length of flash obtained from a typical venous entry may not be sufficient for blood flow to be observed in the flexible tubing at a location proximal to the IV needle assembly itself. Thus, a medical practitioner may have a delayed indication of venous or arterial access and may incorrectly assume that the blood vessel was not accessed properly. In these situations, the medical practitioner may try to access the blood vessel again even though the initial access was successful. Accordingly, the patient may be subjected to unnecessary trauma during a repeated attempt to access the targeted blood vessel.

A blood collection set corresponding to the first part of claim 1 is disclosed in EP 1 442 705 Al. This blood collection set generally corresponds to Figs. 1A, 1B and Fig. 3, but has in addition in the second hub a venting plug that permits an outflow of air while blocking an outflow of blood or other fluids. This venting mechanism enables air that had existed in interior portions of the blood collection set to be vented, and avoids the need to employ a discard tube.

U.S. 6,261,263 B1 discloses a hub of an arterial punction needle having a tubular and transparent viewing chamber which is accessible to the reflux of blood. The viewing chamber is surrounding by a reserve chamber which is leaktight to ambient air and in air communication with an outlet end of the viewing chamber.

WO 2004/062499 A1 discloses a flashback blood collection needle having an inlet cannula, an outlet cannula and a housing between both cannulae. The outlet cannula extends through a flashback chamber of the housing and the ends of the cannula are positioned adjacent to each other, wherein the outlet cannula extends through the flashback chamber.

### SUMMARY OF THE INVENTION

It is an object of the invention to provide a blood collection set that increases the length of flash such that initial blood flow will be observable in the flexible tubing proximal the IV needle assembly and associated shielding elements.

The blood collection set of the present invention is defined by claim 1.

The invention relates to a blood collection set with an internal chamber provided to increase the internal volume of the blood collection set when compared to a prior art blood collection set which does not have an internal chamber. In this manner, improved flash visualization, can be provided.

"Internal chamber" as used herein means an enclosed space or compartment, which is in communication with the fluid passage of the blood collection set. The "fluid passage" is defined as any part of the blood collection set in which blood flows and can include the lumen through the IV cannula, the passage through the IV hub, the passage through the flexible tubing, the passage through the non-patient hub and lumen through the non-patient cannula. "Flash" is defined as the initial blood flow into the fluid passage on venous entry, which provides an indication of venous or arterial access before an evacuated tube is urged into the open proximal end of the blood collection tube holder. "Flash length" is the linear distance that blood flows in a proximal direction along the fluid passage on venous entry. "Flash volume" is the volume of blood that flows into the fluid passage on venous entry expressed as a percentage of the total internal volume of the blood collection set.

In one embodiment, the blood collection set includes an IV needle assembly, a length of flexible plastic tubing extending from the IV needle assembly and a non-patient needle assembly. The internal chamber is advantageously disposed on or near the non-patient needle assembly to permit an increase in the internal volume of the blood collection set without being located directly in the blood flow path.

The IV needle assembly typically comprises an IV hub having a proximal end, a distal end and a passage extending between the ends. The IV needle assembly typically further comprises an IV cannula having a proximal end mounted in the passage of the IV hub, a pointed distal end projecting distally from the IV hub and a lumen that communicates with the passage through the IV hub. The flexible tubing is typically connected to the proximal end of the IV hub. The IV needle assembly typically includes a packaging cover that protectively encloses the IV needle cannula prior to use. The packaging cover is removed immediately prior to use to permit access to the IV cannula. The IV needle assembly may further include a protective shield that is moveable relative to the IV cannula from an open position where the IV cannula is exposed to a closed position where the IV cannula is substantially shielded. The shield protects against accidental sticks with the used IV cannula. A pair of flexible wings may be mounted to the IV hub or to the shield to facilitate manipulation of the IV needle assembly.

In this embodiment, the non-patient needle assembly includes a non-patient hub having a proximal end and a distal end. The non-patient needle assembly further includes a non-patient cannula having a distal end securely mounted in the hub, a proximal end projecting proximally from the non-patient hub and a lumen that communicates with the passage through the non-patient hub. A multiple sample sleeve is typically mounted over the non-patient cannula and secured to the proximal end of the non-patient hub. External portions of the non-patient hub near the proximal end thereof may be formed with an array of external threads or other mounting structure to enable the non-patient needle assembly to be mounted to a collection tube holder or other such medical device. Or, the holder may be pre-attached with the non-patient needle assembly. The blood collection set may further include a fitting mounted to the proximal end of the flexible plastic tubing and configured for mating with the distal end of the non-patient hub. For example, the fitting may be a female luer fitting that can be engaged with the male luer taper at the distal end of the non-patient hub.

In one embodiment, the internal chamber is located in the non-patient assembly beyond the non-patient cannula proximal end, which means that the air passes through the non-patient cannula proximal end from which blood is drawn, and then into the internal chamber. Specifically, on venous entry air is compressed and flows from the fluid passage and out of the non-patient cannula proximal end where it further flows through the space between needle exterior and multiple sample sleeve. The air then flows into the internal chamber, which may be at the non-patient barb, the non-patient hub thread, the non-patient hub body, or other location or combination of locations that are beyond the non-patient cannula proximal end. The collection tube, which is applied at the non-patient cannula proximal end, draws blood from only the fluid passage and not from the internal chamber.

In another embodiment, a one-way check valve is located within the internal chamber. The valve allows air and blood to enter the internal chamber but shuts closed both when vacuum is applied. Thus, when an evacuated collection tube is applied at the non-patient needle tip, the tube draws blood from the fluid passage but not from the internal chamber, and upon removal of the IV needle from the targeted blood vessel, the check valve closes and inhibits blood from dripping from the IV needle tip.

In a further embodiment, the internal chamber utilizes a branch in the fluid passage, e.g., a "Y" or "T". The branching may be at any location or locations along the fluid passage, but is advantageously at the proximal end such as at the non-patient hub. The branching may be in the form of a separate component added into the fluid passage such as in between the female and male luer fittings or it may be integral within the hub. The branching includes some type of internal chamber as discussed herein.

### BRIEF DESCRIPTION OF THE DRAWINGS

FIG. 1A is a perspective view of a prior art blood collection set and collection tube holder.
FIG. 1B is a top plan view of the blood collection set and collection tube holder shown in FIG. 1A.
FIG. 2 is an exploded perspective view of a prior art blood collection set.
FIG. 3 is side elevation view of a prior art non-patient needle assembly, partly in section.
FIG. 4 is a cross-sectional side view of an embodiment of the non-patient needle assembly of the blood collection set.
FIG. 5 is a perspective view of the same embodiment shown in FIG. 4
FIG. 6 is a cross-sectional side view of an embodiment of the non-patient needle assembly of the blood collection set.
FIG. 7 is a perspective view of the same embodiment shown in FIG. 6
FIG. 8 is a cross-sectional side view of an embodiment of the non-patient needle assembly of the blood collection set.
FIG. 9 is an expanded view of the same embodiment shown in FIG. 8
FIG. 10 is a perspective view of one embodiment of a one way check valve.
FIG. 11 is a plan view of the same embodiment shown in FIG. 10
FIG. 12 is a cross-sectional side view of the same embodiment shown in FIG. 10
FIG. 13 is a perspective view of one embodiment of the blood collection set.
FIG. 14 is a perspective view of one embodiment of the blood collection set.

### DETAILED DESCRIPTION

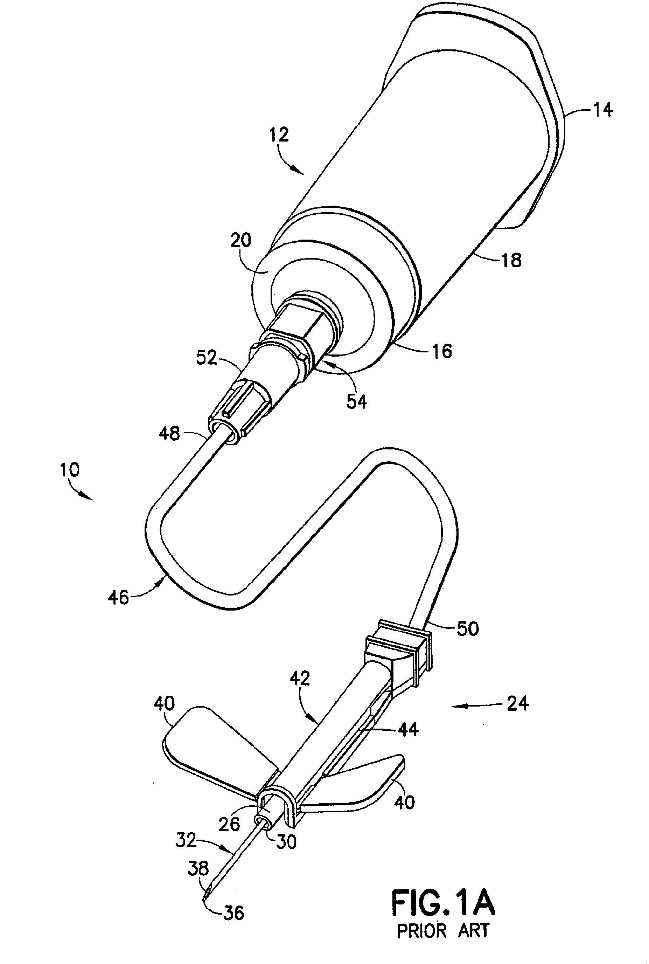

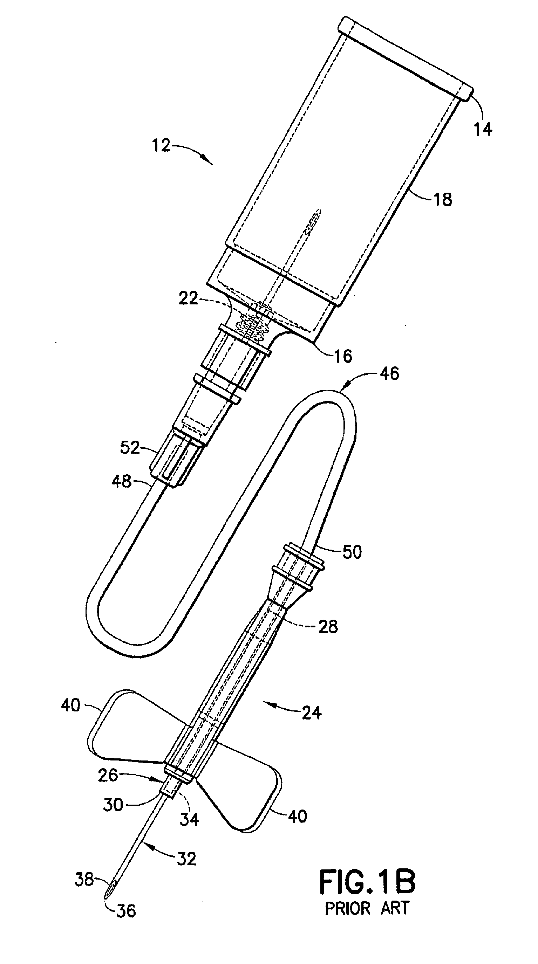

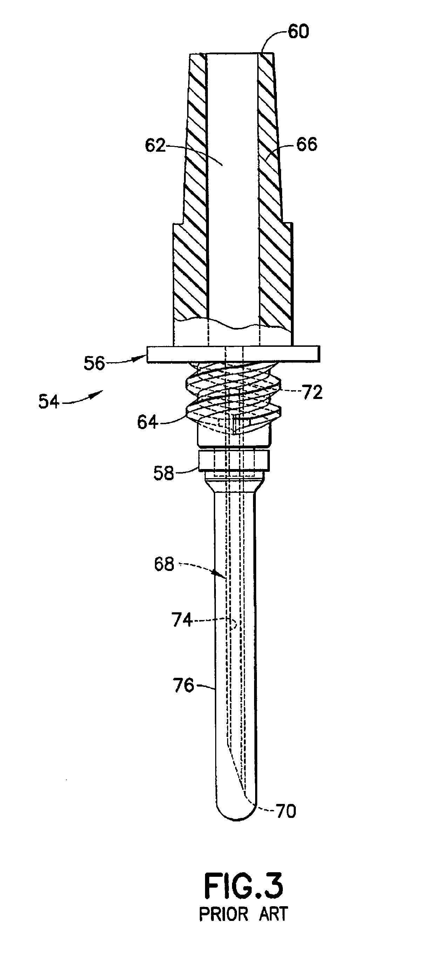

A prior art blood collection set that contains IV cannula shielding is identified generally by the numeral **10** in FIGS. 1A and 1B. Blood collection set **10** is employed in this embodiment with a collection tube holder **12.** Holder **12** has a proximal end **14,** a distal end **16** and a tubular sidewall **18** extending between the ends. Proximal end **14** of holder **12** is widely open and defines an entry to a tube receptacle within sidewall **18.** Thus, an evacuated collection tube can be slid in a proximal-to-distal direction through open proximal end **14** of holder **12** toward distal end **16.** Distal end **16** of holder **12** is characterized by an end wall **20.** End wall **20** is formed with an internally threaded mounting aperture **22,** as shown in FIG. 3.

Blood collection set **10** includes an IV needle assembly **24** that comprises an IV hub **26.** IV hub **26** includes a proximal end **28,** a distal end **30** and a passage (not shown) extending between the ends. IV needle assembly **24** further includes an IV cannula **32** with a proximal end **34,** a pointed distal end **36** and a lumen **38** extending between the ends. Proximal end **34** of IV cannula **32** is mounted securely in the passage of IV hub **26.** Thus, lumen **38** through IV cannula **32** communicates with the passage through IV hub **26.** Flexible wings **40** are mounted to IV hub **26** at a location near distal end **30.** Wings **40** can be folded into face-to-face relationship with one another for convenient gripping between a thumb and forefinger to enable manipulation of IV needle assembly **24.** Wings **40,** however, also can be rotated into a substantially coplanar disposition for taping to the skin of a patient.

IV needle assembly **24** further includes a tubular shield **42** that is telescoped over IV hub **26.** Shield **42** is formed with transverse slots **44** that slidably receive wings **40.** Thus, shield **42** can be slid from a proximal position, as shown in FIGS. 1A and 1B to a distal position. IV cannula **32** is exposed for use when shield **42** is in the proximal position shown in FIGS. 1A and 1B. However, IV cannula **32** is substantially surrounded by shield **42** when shield **42** is moved to the distal position. Additionally, slots **44** in shield **42** are configured to lockingly engage wings **40** when shield **42** is in the distal position to prevent or complicate a re-exposure of IV cannula **32.** In this case the presence of a shield does not inhibit flash visualization within the IV needle assembly. The shield illustrated in FIGS. 1A and 1B is one of many optional shield designs that can be incorporated into blood collection set **10.** Other designs may provide wings mounted directly to the shield. Still other designs may provide a hinged shield mounted to IV hub **26.** In still other designs, a shield may be entirely separate from IV needle assembly **24.**

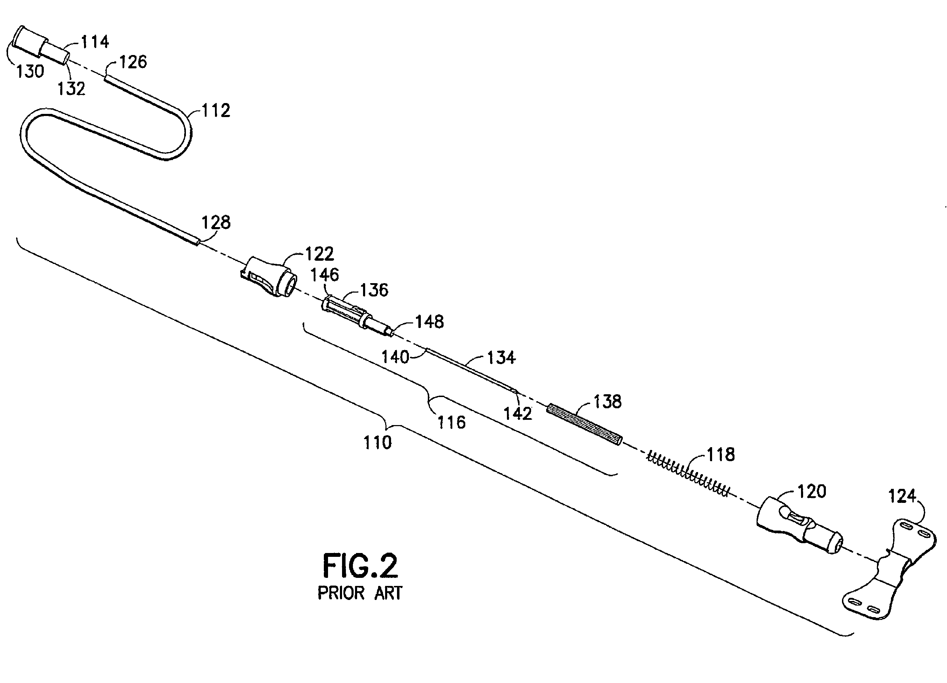

Another prior art blood collection set is identified generally by the numeral **110** in FIG. 2. Fluid collection/infusion set **110** includes a length of flexible plastic tubing **112,** a proximal fitting **114,** a needle assembly **116,** a spring **118** and a barrel assembly that comprises a front barrel **120,** a rear barrel **122** and a wing **124.** Needle assembly **116** includes a needle cannula **134,** a needle hub **136** and a needle protector **138.** Needle cannula **134** has a proximal end **140,** a distal end **142** and a lumen **144** extending between the ends. Distal end **142** of needle cannula **134** is beveled to a sharp tip.

FIGS. 1A and 1B also show that a blood collection set **10** further includes a length of flexible plastic tubing **46.** Tubing **46** includes opposite proximal and distal ends **48** and **50** and a passage extending between the ends. Distal end **50** of tubing **46** is securely mounted to proximal end **28** of IV hub **26** so that the passage through IV hub **26** communicates with the passage through tubing **46.** A female luer fitting **52** is securely mounted to proximal end **48** of tubing **46.**

Blood collection set **10** further includes a non-patient needle assembly **54,** as shown in FIG. 3. Non-patient needle assembly **54** includes a non-patient hub **56** with a proximal end **58,** a distal end **60** and a fluid passage **62** extending between the ends. Exterior surface regions of non-patient hub **56** substantially adjacent proximal end **58** define an array of external threads **64** configured for threaded engagement with the internal threads formed in mounting aperture **22** of collection tube holder **12.** External surface regions of non-patient hub **56** adjacent distal end define a male luer taper **66** configured for mating with female luer fitting **52.** Non-patient needle assembly **54** further includes a non-patient cannula **68** having a pointed proximal end **70,** a distal end **72** and a lumen **74** extending between the ends. Distal end **72** of non-patient cannula **68** is mounted securely in passage **62** through non-patient hub **56** and aligns substantially with external threads **64** on non-patient hub **56.** Non-patient needle assembly **54** further includes a multiple sample sleeve **76** mounted over non-patient cannula **68** and securely engaged with proximal end **58** of non-patient hub **56.** Multiple sample sleeve **76** effectively functions as a valve that prevents a flow of fluid from non-patient cannula **68.** However, multiple sample sleeve **76** can be pierced by pointed proximal end **70** of non-patient cannula **68** in response to forces generated by a stopper on an evacuated collection tube.

It should be noted that flash visualization maybe acceptable in certain blood collection sets with safety features, such as the type shown in FIGS. 1A and 1B. The IV needle assembly and safety shield tend to be made from a translucent plastic material, and may only contain the IV cannula and flexible tubing, which may allow flash to be seen through the IV cannula hub walls. Therefore a minimum length of flash maybe sufficient, such that initial blood flow on venous entry maybe observed at the proximal end of the IV cannula. However in more advanced and complex IV cannula shielding mechanisms, such as the blood collection set shown in FIG. 2, the length of flash obtained from a typical venous entry may not be sufficient for blood flow to be observed in the flexible tubing at a location proximal to the IV needle assembly itself, therefore flash visualization tends to be inhibited within the IV needle assembly.

According to one embodiment of the invention, a blood collection set, for example of the type disclosed above, contains an internal chamber that increases the internal volume of the blood collection set. The increase in internal volume of the blood collection set provides a greater length of flash and therefore improved flash visualization, such that, advantageously, blood is observed in the flexible tubing proximal the IV needle assembly on the initial venous entry. While such improved or lengthened flash is particularly advantageous for such complex blood collection sets as shown in FIG 2, application in any blood collection set is contemplated.

According to this embodiment of the invention, a blood collection set includes a plurality of internal spaces that will initially be at ambient air pressure. These internal spaces include the lumen through the IV cannula, the passage through the IV hub, the passage through the flexible tubing, the passage through the non-patient hub and lumen through the non-patient cannula. The blood collection set is employed by folding wings into face-to-face engagement with one another and gripping the wings between a thumb and forefinger. Any packaging cover that may be mounted over IV cannula then is removed and discarded. The pointed distal end of the IV cannula then is urged into a targeted blood vessel. The healthcare practitioner then may release the grip on the wings, and if long term access to the blood vessel is required, the wings may be taped into face-to-face engagement with the skin of the patient. The venous or arterial access achieved with the IV cannula places the plurality of interior spaces of the blood collection set in communication with the pressure of the blood in the patient. Blood pressure exceeds the ambient air pressure. Accordingly, the pressure of air in the above-referenced internal spaces will increase, and blood will begin to flow into these internal spaces. The system will reach equilibrium as the air pressure within the blood collection set increases in response to a reduction of volume caused by the inflow of blood. Hence, a portion of the internal spaces in the system will remain filled with air at a pressure substantially equal to the venous or arterial pressure thereby preventing blood from flowing any further to or into the tubing. Stated differently, the system will include its original volume of air in the space between the proximal end of the non-patient needle and the blood that enters the blood collection set. This pressurized air will prevent any further flow of blood toward the non-patient assembly, thereby stopping flash. In prior art systems the total volume of the internal spaces maybe insufficient, such that flash maybe stopped before it can be observed. According to embodiments of the invention, however, the internal chamber which lacks direct communication to the exterior, provides more internal volume into which the air can be pushed, allowing blood to push further into the device before the pressure of the blood equals the pressure of the air. This increase in interior space thereby allows a greater length of flash to occur. Various embodiments of such internal chambers are described in detail below.

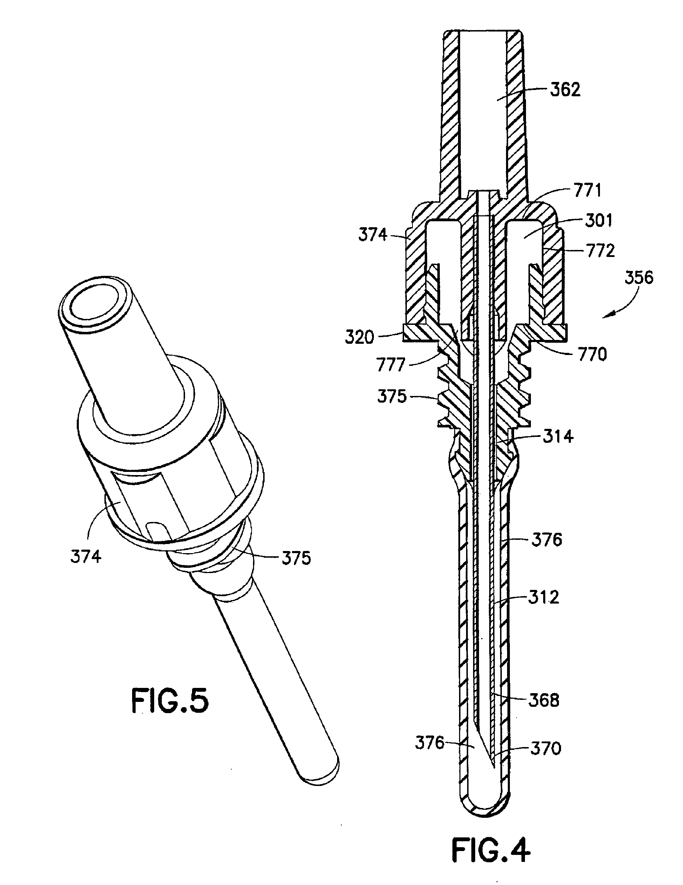

FIGS. 4 and 5 show an embodiment of the invention, where the internal chamber **301** is located beyond the proximal end **370** of the non-patient cannula in the non-patient hub **356** and lacks direct communication to the exterior. The internal chamber **301** is annular in cross-section and has opposite proximal **770** and distal ends **771** and a wall **772** extending therebetween; the proximal end **770** has an inlet **777** in communication with the passage. However all internal chamber geometries can be considered for the invention. The non-patient hub **356** is generally made from 2 separate parts; the male luer **374** and the non-patient thread assembly **375.** Other configurations are possible. On venous entry, air is compressed and flows from the fluid passage **362** and out of the non-patient cannula proximal end **370** where it further flows through the space **312** between needle exterior **368** and multiple sample sleeve interior **376,** then through a passage **314** through the non-patient barb and the male luer wall **320** into the internal chamber **301** through the inlet **777** in the male luer **374.**

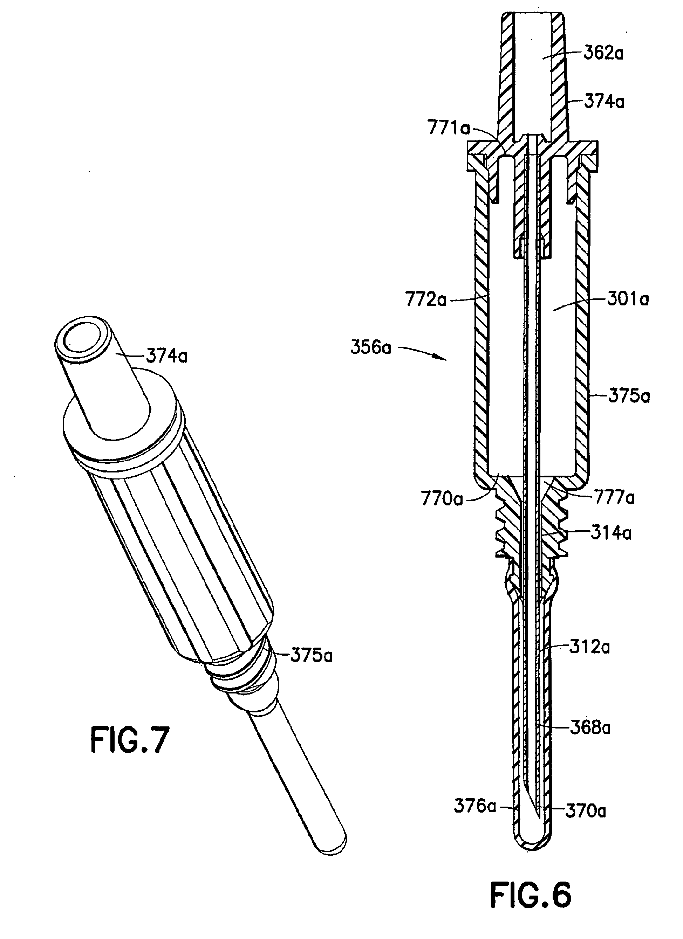

FIGS 6 and 7 show another embodiment of the invention, with an elongated internal chamber to further increase the flash length. The internal chamber **301a** is annular in cross-section and has opposite proximal **770a** and distal ends **771a** and a wall **772a** extending therebetween, the proximal end **770a** has an inlet **777a** in communication with the passage. However all internal chamber geometries can be considered for the invention. The non-patient hub is made from 2 separate parts; the male luer **374a** and the non-patient thread assembly **375a.** The longer internal chamber **301a** is located beyond the proximal end **370a** of the non-patient cannula in the non-patient thread assembly **375a** and has no direct communication to the exterior. Other configurations are possible. On venous entry, air is compressed and flows from the fluid passage **362a** and out of the non-patient cannula proximal end **370a** where it further flows through the space **312a** between needle exterior **368a** and multiple sample sleeve interior **376a,** then through a passage **314a** through the non-patient barb into an internal chamber **301a** through an inlet **777a** in the non-patient thread assembly **375a.**

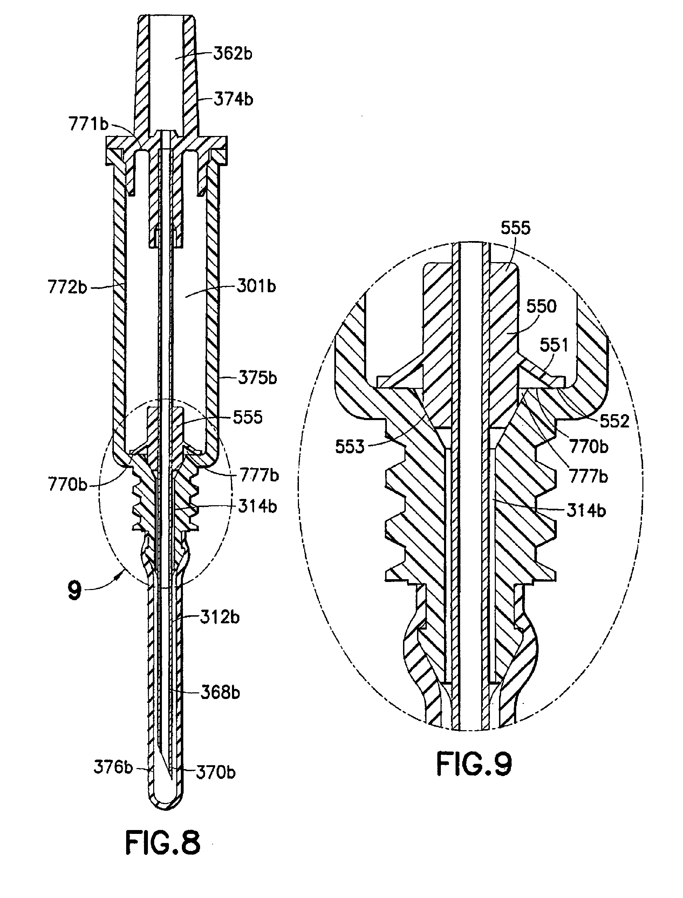

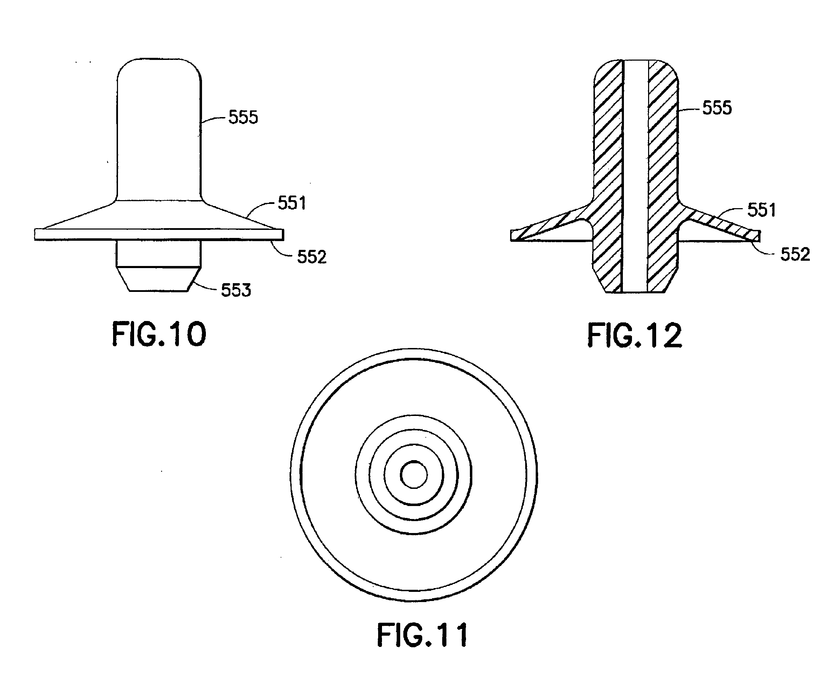

FIGS 8 to 12 show a further embodiment of the invention, in which a one-way valve **555** is located within an internal chamber similar to that shown in FIG. 6. The one way valve has a central core **550** surrounded by an annular flange **551** that has a sealing ring **552** around its outer diameter. The central core **550** is located around the non-patient needle exterior **368b** within the internal chamber **301b** and has a tapered base **553** which mates to the taper of inlet **777b.** Typically the one way valve **555** is formed from a resilient polymer such as an elastomer or thermoplastic.

As apparent to one skilled in the art, on venous entry, air flows from the fluid passage **362b** and out of the non-patient cannula proximal end **370b** where it further flows through the space **312b** between needle exterior **368b** and multiple sample sleeve interior **376b,** then through a passage **314b,** through the non-patient barb and the male luer wall **320b,** through the inlet **777b** where it moves the one-way valve **555,** flowing past the valve core base **553,** valve sealing ring **552** and into an internal chamber **301b** in the male luer **374b**. A vacuum is applied to the fluid passage, when an evacuated collection tube is applied at the non-patient needle tip. The resultant positive pressure differential between the internal chamber and the fluid passage causes the one way valve **555** to close, by the valve sealing ring **552** being pressed against the proximal end of the internal chamber **770b** and the valve core tapered base **553** being pressed into inlet 777b thereby preventing outflow of air or blood from the internal chamber,. It should be noted that the one way valve is not limited to the particular design described in this embodiment as all types of one way valves can be contemplated for this invention.

The one way valve **555** also inhibits blood from dripping from the IV needle tip upon removal of the IV needle from the targeted blood vessel after the blood collection has been completed. In prior art blood collection sets, at the instance of removal of the IV needle, the fluid passage of the blood collection set, will contain a reservoir of air and blood at venous pressure, which will be greater than the ambient atmospheric pressure. Thus a back pressure is created within the fluid passage which may cause a back-flow of blood to drip from the IV needle tip. In the embodiment of the invention shown in FIGS 8 to 12, the reservoir of air at venous pressure is within the internal chamber **301b,** therefore the back pressure causes the one way valve **555** to shut upon removal of the IV needle thereby preventing such a back-flow of blood in the fluid passage **362b.**

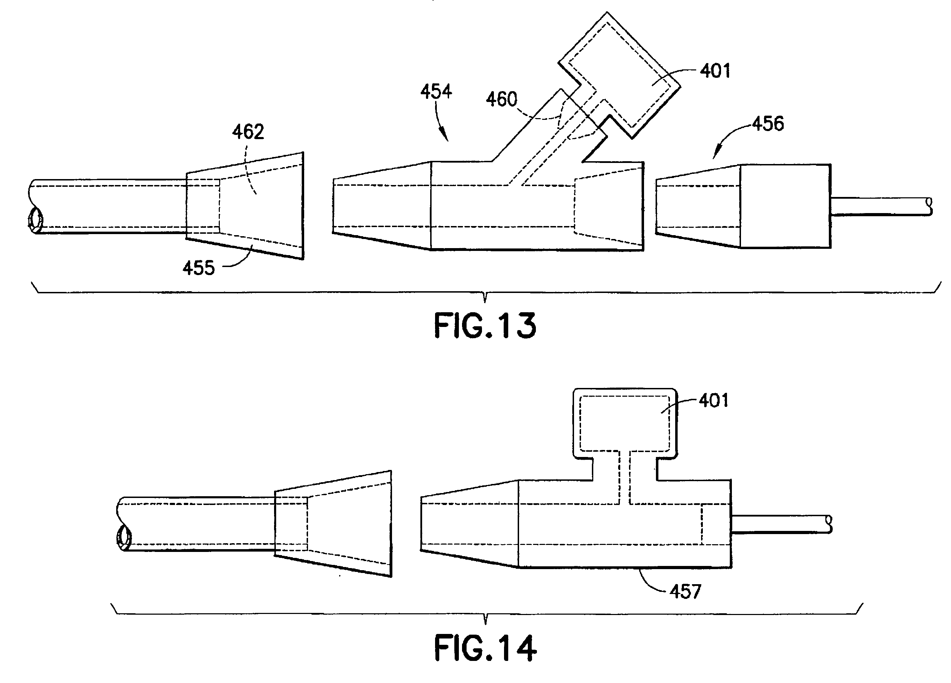

FIGS 13 and 14 show another embodiment of an internal chamber **401** which is branched from the fluid passage using a "Y" or "T" branched internal chamber in the fluid passage into which air is displaced. The branching may be at any location or locations along the fluid passage, but is typically near the proximal end such as at the non-patient hub. FIG 13 shows a "Y" branched internal chamber **454** in the form of a separate component added into the fluid passage **462** such as in between the female **455** and male luer **456** fittings. FIG 14 shows a "T" branched internal chamber **457,** which is an integral part of the non-patient hub, thus reducing the number of components. The use of a "Y" or "T" branched internal chamber may be accomplished with any convenient terminal shapes at the interfaces of each component. The embodiments exemplified previously are shown with luer tapered fittings but other interface designs such as direct connection to the flexible tubing may also be applied. Although typical applications use a three port "Y" or "T" branched internal chamber the use of a multiple port branched internal chamber system is also possible. The internal chamber **401,** can be a separate component which is affixed into a port **460** of the branch system as shown in FIG 13 or an integral part of the branched system as shown in FIG 14. All internal chamber geometries can also be contemplated.

## Claims

1. A blood collection set comprising:
a first needle assembly (24) comprising a first hub (26) and a first cannula (32) having a lumen therethrough, wherein said cannula is mounted to said first hub,
a length of flexible tubing (46) having opposite first and second ends (48, 50) and a tubing passage extending between said ends, said first hub (26) located proximate said first end (50) for providing communication between said lumen of said first cannula and said tubing passage,
a second hub (356) located proximate said second end (48) and having a passage (362) extending therethrough, said second hub passage (362) being in fluid communication with said tubing passage,
a non-patient cannula (368) having opposite proximal (370) and distal ends and a lumen extending between said ends, said distal end of said non-patient cannula being mounted to said second hub (356) such that said lumen through said non-patient cannula communicates with said second hub passage (362),
a multiple sample sleeve (376) covering said non-patient cannula (368) and mounted to said second hub,
**characterized by**
an internal chamber (301) in communication with said second hub passage (362), wherein said internal chamber is located within said second hub (356) or is branched from said second hub passage (362), and wherein said internal chamber (301) lacks direct communication to the exterior,
such that on venous entry air is compressed and flows through the non-patient cannula proximal end and then into the internal chamber.

2. The blood collection set of claim 1, wherein said second hub (356) further comprises a male luer and a non-patient thread assembly(375), which form said internal chamber (301).

3. The blood collection set of claim 1, wherein said internal chamber (301) communicates with said second hub passage (362) beyond said proximal end of said non-patient cannula.

4. The blood collection set of claim 1, further comprising a one way valve (555), wherein said one way valve inhibits an outflow of air or fluid from said internal chamber (301b).

5. The blood collection set of claim 4, wherein said one way valve (555) is located within said internal chamber (301b).

6. The blood collection set of claim 1, wherein upon venous access by said first needle cannula (32), venous pressure causes blood to flow into said tubing passage, such that the total internal volume of said internal chamber (301) and tubing passage results in a flash length of at least approximately 0.635 cm (0.25 inches) in linear distance in a proximal direction along said tubular passage from said first end (50) of said flexible tubing (46).

7. The blood collection set of claim 1, wherein upon venous access by said first needle cannula venous pressure causes blood to flow into said tubing passage, such that the total internal volume of said internal chamber (301) and tubing passage results in a flash length of at least approximately 1,27 cm in linear distance in a proximal direction along said tubular passage from said first end (50) of said flexible tubing (46).

8. The blood collection set of claim 1, wherein said internal chamber (401) is branched from said second hub passage (462).

9. The blood collection set of claim 8, wherein said internal chamber (401) is a Y branched internal chamber or a T branched internal chamber.

10. The blood collection set of claim 1, wherein the volume of said internal chamber is at least approximately 1,0 ml.

11. The blood collection set of claim 10, wherein said volume of said internal chamber is approximately 1,0 ml to approximately 3,0 ml.

12. The blood collection set of claim 11, wherein said volume of said internal chamber is approximately 1,0 ml to approximately 2,0 ml.

13. The blood collection set of one of claims 1 - 12, wherein a flash resulting from a blood flow into the total internal volume of the internal chamber (301) and tubing chamber can be observed within said flexible tubing (46).

## Patentansprüche

1. Blutsammelset mit:
einer ersten Nadelanordnung (24) mit einem ersten Ansatz (26) und einer ersten Kanüle (32) mit einem durch diese hindurchgehenden Lumen, wobei die Kanüle an dem ersten Ansatz angebracht ist,
einem Stück eines biegsamen Schlauchs (46) mit entgegengesetzten ersten und zweiten Enden (48, 50) und einem sich zwischen den Enden erstreckenden Schlauchdurchlass, wobei der erste Ansatz (26) nahe dem ersten Ende (50) angeordnet ist, um eine Verbindung zwischen dem Lumen der ersten Kanüle und dem Schlauchdurchlass herzustellen,
einem zweiten Ansatz (356), der sich nahe dem zweiten Ende (48) befindet und einen durchgehenden Durchlass (362) aufweist, wobei der Durchlass (362) des zweiten Ansatzes in Fluidverbindung mit dem Schlauchdurchlass steht,
einer Nichtpatientenkanüle (368) mit entgegengesetzten proximalen (370) und distalen Enden und einem sich zwischen den Enden erstreckenden Lumen, wobei das distale Ende der Nichtpatientenkanüle an dem zweiten Ansatz (356) derart angebracht ist, dass das durch die Nichtpatientenkanüle hindurchgehende Lumen in Verbindung mit dem Durchlass (362) des zweiten Ansatzes steht,
einer Mehrfachprobenhülse (376), welche die Nichtpatientenkanüle (368) abdeckt und an dem Ansatz angebracht ist,
**gekennzeichnet durch**
eine mit dem Durchlass (362) des zweiten Ansatzes verbundene innere Kammer (301), wobei die innere Kammer in dem zweiten Ansatz angeordnet ist oder von dem Durchlass (362) des zweiten Ansatzes abzweigt, und wobei die innere Kammer (301) nicht unmittelbar mit der mit der äußeren Umgebung verbunden ist, so dass beim Veneneintritt Luft komprimiert wird und **durch** das proximale Ende der Nichtpatientenkanüle und anschließend in die innere Kammer strömt.

2. Blutsammelset nach Anspruch 1, bei welcher der zweite Ansatz (356) ferner einen männlichen Luer-Verbinder und einer Nichtpatientengewindeanordnung (375) aufweist, welche die innere Kamme (301) bilden.

3. Blutsammelset nach Anspruch 1, bei welcher die innere Kammer (301) über das proximale Ende der Nichtpatientenkanüle hinaus in Verbindung mit dem Durchlass (362) des zweiten Ansatzes steht.

4. Blutsammelset nach Anspruch 1, ferner mit einem Einwegventil (555), wobei das Einwegventil das Ausströmen von Luft oder Fluid aus der inneren Kammer (301b) verhindert.

5. Blutsammelset nach Anspruch 4, bei welchem das Einwegventil (555) sich innerhalb der inneren Kammer (301b) befindet.

6. Blutsammelset nach Anspruch 1, bei welchem beim Veneneintritt der ersten Nadelkanüle (32) der Venendruck das Fließen von Blut in den Schlauchdurchlass bewirkt, so dass das Gesamt-Innenvolumen der inneren Kammer (301) und des Schlauchdurchlasses zu einer Flash-Länge führt, die linear in proximaler Richtung entlang des schlauchförmigen Durchlasses von dem ersten Ende (50) des biegsamen Schlauchs (46) aus mindestens ungefähr 0,635 cm (0,25 Inch) beträgt.

7. Blutsammelset nach Anspruch 1, bei welchem beim Veneneintritt der ersten Nadelkanüle der Venendruck das Fließen von Blut in den Schlauchdurchlass bewirkt, so dass das Gesamt-Innenvolumen der inneren Kammer (301) und des Schlauchdurchlasses zu einer Flash-Länge führt, die linear in proximaler Richtung entlang des schlauchförmigen Durchlasses von dem ersten Ende (50) des biegsamen Schlauchs (46) aus mindestens ungefähr 1,27 cm beträgt.

8. Blutsammelset nach Anspruch 1, bei welchem die innere Kammer (401) von dem Durchlass (462) des zweiten Ansatzes abgezweigt ist.

9. Blutsammelset nach Anspruch 8, bei welchem die innere Kammer (401) eine Y-förmig abgezweigte innere Kammer oder eine T-förmig abgezweigte innere Kammer ist.

10. Blutsammelset nach Anspruch 1, bei welchem das Volumen der inneren Kammer mindestens ungefähr 1,0 ml beträgt.

11. Blutsammelset nach Anspruch 10, bei welchem das Volumen der inneren Kammer ungefähr 1,0 ml bis ungefähr 3,0 ml beträgt.

12. Blutsammelset nach Anspruch 11, bei welchem das Volumen der inneren Kammer ungefähr 1,0 ml bis ungefähr 2,0 ml beträgt.

13. Blutsammelset nach einem der Ansprüche 1-12, bei welchem ein Flash, der durch einen Blutstrom in das Gesamt-Innenvolumen der inneren Kammer (301) und der Schlauchkammer hervorgerufen wird, in dem flexiblen Schlauch (46) beobachtet werden kann.

## Revendications

1. Ensemble de collecte de sang comprenant:
un premier ensemble d'aiguille (24) comprenant un premier embout (26) et une première canule (32) avec une lumière à travers la même, la canule étant montée sur ledit premier embout,
une pièce de tuyau flexible (46) comprenant une première et une deuxième extrémité (48, 50), opposées l'une à l'autre, et une passage de tuyau s'étendant entre lesdites extrémités, ledit premier embout (26) étant situé près de ladite première extrémité (50) pour établir la communication entre ladite lumière de la première canule et ladite passage de tuyau,
un deuxième embout (356) situé près de ladite deuxième extrémité (48) et comprenant une passage (362) s'étendant à travers le même, ladite passage (362) du deuxième embout étant en communication fluidique avec ladite passage de tuyau,
une canule non-patient (368) comprenant une extrémité proximale (370) et une extrémité distale, opposées l'une à l'autre, et une lumière s'étendant entre lesdites extrémités, l'extrémité distale de ladite canule non-patient étant montée sur ledit deuxième embout (356) de manière que la lumière à travers ladite canule non-patient est en communication avec ladite passage (362) du deuxième embout,
une manche de prélèvement multiple (376) recouvrant ladite canule non-patient (368) et montée sur ledit deuxième embout,
**caractérisé par**
une chambre interne (301) en communication avec ladite passage (362) dudit deuxième embout, ladite chambre interne étant située dans ledit embout (356) ou branchée de ladite passage (362) dudit deuxième embout, et la chambre interne (301) manquant de communication directe avec l'extérieur de manière que, lorsqu'on entre la veine, de l'air est comprimé et s'écoule à travers ladite extrémité proximale de la canule non-patient et après dans ladite chambre interne.

2. Ensemble de collecte de sang selon la revendication 1, dans lequel ledit deuxième embout (356) en outre comprend un connecteur mâle du type Luer et un ensemble de filetage non-patient (375), format ladite chambre interne (301).

3. Ensemble de collecte de sang selon la revendication 1, dans lequel ladite chambre interne (301) est en communication avec ladite passage (362) dudit deuxième embout au-delà de ladite extrémité proximale de la canule non-patient.

4. Ensemble de collecte de sang selon la revendication 1, en outre comprenant une valve unidirectionnelle (555), ladite valve unidirectionnelle empêchant la fuite de l'air ou de fluide de ladite chambre interne (301b).

5. Ensemble de collecte de sang selon la revendication 4, dans lequel ladite valve unidirectionnelle (555) est située dans ladite chambre interne (301b).

6. Ensemble de collecte de sang selon la revendication 1, dans lequel, lorsque ladite première canule (32) entre la veine, la pression veineuse cause l'écoulement du sang dans ladite passage de tuyau, de manière que le volume interne total de ladite chambre interne (301) et de ladite passage de tuyau résulte en une longueur de flash d'environ au moins 0,635 cm (0,25 Inch), écartée linéairement de ladite première extrémité (50) dudit tuyau flexible (46) selon la direction proximale le long de ladite passage tubulaire.

7. Ensemble de collecte de sang selon la revendication 1, dans lequel, lorsque ladite première canule entre la veine, la pression veineuse cause l'écoulement du sang dans ladite passage de tuyau, de manière que le volume interne total de ladite chambre interne (301) et de ladite passage de tuyau résulte en une longueur de flash d'environ au moins 1,27 cm, écartée linéairement de ladite première extrémité (50) dudit tuyau flexible (46) selon la direction proximale le long de ladite passage tubulaire.

8. Ensemble de collecte de sang selon la revendication 1, dans lequel ladite chambre interne (401) est branchée de ladite passage (462) dudit deuxième embout.

9. Ensemble de collecte de sang selon la revendication 8, dans lequel ladite chambre interne (401) est une chambre interne branchée en Y ou une chambre interne branchée en T.

10. Ensemble de collecte de sang selon la revendication 1, dans lequel le volume de ladite chambre interne est au moins environ 1,0 ml.

11. Ensemble de collecte de sang selon la revendication 10, dans lequel le volume de ladite chambre interne est entre environ 1,0 ml et environ 3,0 ml.

12. Ensemble de collecte de sang selon la revendication 11, dans lequel le volume de ladite chambre interne est entre environ 1,0 ml et environ 2,0 ml.

13. Ensemble de collecte de sang selon l'une quelconque des revendications 1-12, dans lequel un flash résultant de l'écoulement de sang dans le volume interne total de ladite chambre interne (301) et la chambre de tuyau peut être observé dans ledit tuyau flexible (46).
